(19) Europäisches Patentamt
European Patent Office
Office européen des brevets

(11) EP 0 951 896 B1

(12) **EUROPÄISCHE PATENTSCHRIFT**

(45) Veröffentlichungstag und Bekanntmachung des Hinweises auf die Patenterteilung:
**21.12.2005 Patentblatt 2005/51**

(51) Int Cl.[7]: **A61K 6/083**

(21) Anmeldenummer: **99250101.5**

(22) Anmeldetag: **31.03.1999**

(54) **Radikalisch polymerisierbarer Dentalwerkstoff**

Radically polymerizable dental material

matériel dentaire à polymérisation radicalaire

(84) Benannte Vertragsstaaten:
**AT CH DE FR GB IT LI SE**

(30) Priorität: **24.04.1998 DE 19818210**

(43) Veröffentlichungstag der Anmeldung:
**27.10.1999 Patentblatt 1999/43**

(73) Patentinhaber: **Ivoclar Vivadent AG**
**9494 Schaan (LI)**

(72) Erfinder:
- **Rumphorst, André, Dr.**
**9490 Vaduz (LI)**
- **Salz, Ulrich, Dr.**
**88131 Lindau (DE)**
- **Gianasmidis, Alexandros**
**9435 Heerbrugg (CH)**
- **Völkel, Thomas, Dr.**
**88131 Lindau (DE)**
- **Moszner, Norbert, Prof. Dr.**
**FL-9495 Triesen (LI)**
- **Rheinberger, Volker, Dr.**
**9490 Vaduz (LI)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 491 169**
**DE-A- 4 104 934**
**US-A- 3 940 362**
**US-A- 5 710 194**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft zwei- und mehrkomponentige, radikalisch polymerisierbare Dentalwerkstoffe, die sich insbesondere als Zemente und Füllungsmaterialien eignen.

Zemente und Füllungsmaterialien lassen sich grob in Glasionomerzemente und Komposite unterscheiden (vgl. beispielsweise R. Hickel, Stomatologie (1997) 94/7:363-382). Glasionomerzemente sind wasserhaltige, zweikomponentige Zemente auf Basis polymerer organischer Säuren wie beispielsweise Poly(acrylsäure) und pulverförmigen, festen Basen wie Calcium-Fluor-Aluminiumsilikat-Gläsern. Die Aushärtung des Zements erfolgt durch ionische Reaktion zwischen polymergebundenen Carboxylgruppen und aus dem Füllstoff austretenden Calcium- oder Aluminiumionen. Glasionomerzemente zeichnen sich durch eine hohe Fluoridabgabe aus, welche die Gefahr von Sekundärkaries reduziert, eignen sich aufgrund ihrer schlechten Biegefestigkeit jedoch nicht für occlusionstragende Füllungen.

Als Komposite werden Zusammensetzungen bezeichnet, die im wesentlichen aus einem polymerisierbaren Bindemittel und einem organischen oder anorganischen Füllstoff bestehen. Komposite weisen eine deutlich höhere Biegefestigkeit als Glasionomerzemente auf, setzen jedoch im allgemeinen keine Fluoridionen frei und erfordern zudem den Einsatz von Dentinadhäsiven und absolut trockene Bedingungen bei der Anwendung.

Neuerdings wird versucht, die positiven Eigenschaften von Glasionomerzementen und Kompositen zu vereinigen. Hierzu wurden wasserhaltige, kunststoffmodifizierte Glasionomerzemente (Hybridionomere) und wasserfreie Compomere vorgeschlagen, deren Härtung sowohl auf einer Säure-Base-Reaktion als auch auf einer radikalischen Polymerisation basiert.

Die Härtung von Kompositen, Hybridionomeren und Compomeren umfaßt in der Regel eine radikalische Polymerisation, die chemisch, durch Licht oder Wärme initiiert wird. Die Licht- oder Wärmehärtung hat den Vorteil, daß die Materialien bis zur Bestrahlung mit einer geeigneten Lichtquelle verarbeitbar bleiben und bei Lagerung in lichtundurchlässigen Behältern in der Regel über lange Zeiträume haltbar sind. Die Verwendung von Photoinitiatoren ist jedoch auf lichtdurchlässige Materialien beschränkt und bei tiefen Kavitäten ist eine schrittweise Verarbeitung und Härtung erforderlich. Außerdem ist der Einsatz von photopolymerisierbaren Materialien auf Bereiche beschränkt, die für die Polymerisationslampe zugänglich sind. Wärmehärtbare Materialien sind im Munde des Patienten nicht einsetzbar.

Die chemische Härtung erfolgt durch die Verwendung von Redoxsystemen als Polymerisationsinitiatoren. Diese haben den Vorteil, daß sie auch bei opaken Materialien und tiefen Kavitäten anwendbar sind. Nachteilig ist jedoch ihre häufig nur beschränkte Lagerbeständigkeit.

Redox-Initiator-Systeme zur chemischen Polymerisation enthalten Initiatoren wie Peroxide oder Azoverbindungen, die freie Radikale bilden können (oxidierender Stoff) und einen als Aktivator dienenden reduzierenden Stoff. Die Polymerisation wird durch das Mischen von Initiator und Aktivator ausgelöst. ,

Die US-A-3,991,008 offenbart polymerisierbare Dentalmaterialien mit verbesserter Farb- und Lagerstabilität, die als Reduktionsmittel substituierte Thioharnstoffderivate enthalten.

Die US-A-5,554,665 beschreibt die Verwendung von sauerstoffdurchlässigen Behältern zur Lagerung von chemisch härtbaren Dentalmaterialien. Durch den eintretenden Luftsauerstoff soll die Polymerisation der Komponenten inhibiert werden.

Die US-A-5,367,002 offenbart Dentalmaterialien, die eine härtbare flüssige Zusammensetzung und eine pulverförmige Komponente enthalten. Die Härtung der Materialien erfolgt einerseits durch die ionische Reaktion von Polyalkensäuren mit einem ionenfreisetzenden Füllstoff und andererseits durch radikalische Polymerisation. Die Werkstoffe können ein Redox-Initiator-System enthalten, wobei die Bestandteile des Redox-Systems auf verschiedene Komponenten des Dentalmaterials verteilt sind. Die Systeme weisen aufgrund hydrolytischer und/oder unerwünschter Redox-Reaktionen in der flüssigen Phase nur eine beschränkte Haltbarkeit auf. Die Verwendung stark saurer Monomere ist aufgrund möglicher Reaktionen mit den Bestandteilen des Redox-Systems nicht möglich.

Die US 3,940,362 offenbart polymerisierbare Adhäsive, die sich besonders für dentale Anwendungen eignen sollen. Die Adhäsive enthalten einen monomeren Ester von $\alpha$-Cyanoacrylsäure, ein difunktionelles, monomeres Vernetzungsmittel, das ein Diester von Acryl- oder Methacrylsäure und einem Alkohol mit mindestens zwei veresterbaren Hydroxylgruppen ist, Füllstoff und Natriumfluorid. Die monomeren Verbindungen der Adhäsive werden durch aminaktivierte, radikalische Initiatoren polymerisiert.

Gemäß der US-A-5 154 762 werden die Bestandteile des Redox-Systems mikroverkapselt, so daß beide Komponenten in die feste Phase eingearbeitet werden können. Auf diese Weise lassen sich Reaktionen in der flüssigen Phase vermeiden und die Haltbarkeit der Materialien steigern. Allerdings müssen zur Initiierung der Polymerisation Initiator und Aktivator durch Auflösen der Mikrokapseln oder durch deren mechanische Zerstörung freigesetzt werden. Dies erfordert entweder eine relativ lange Aktivierungszeit oder den Einsatz hoher mechanischer Kräfte. Außerdem handelt es sich bei der Mikroverkapselung um ein aufwendiges und damit kostenintensives Verfahren.

Aufgabe der vorliegenden Erfindung ist die Bereitstellung eines zwei- oder mehrkomponentigen, bei Raumtemperatur polymerisierbaren Dentalwerkstoffs, bei dem die Bestandteile des Redox-Initiator-Systems in einer festen Komponente enthalten sind, das einfach herstellbar und ohne die beschriebenen Nachteile polymerisierbar ist.

Diese Aufgabe wird durch ein Dentalfüllstoffgemisch sowie Dentalwerkstoffe mit mindestens einem polymerisierbaren Bindemittel und mindestens einem Füllstoffgemisch gelöst, wobei das Füllstoffgemisch ein Redox-Initiator-System für die radikalische Polymerisation enthält, das einen Initiator und einen Aktivator umfaßt herstellbar nach dem Verfahren gemäß Anspruch 1 und 14. Das Verfahren ist dadurch gekennzeichnet, daß man einen ersten Teil des Füllstoffs mit dem Initiator beschichtet, man einen zweiten Teil des Füllstoffs mit dem Aktivator beschichtet, und man den ersten und zweiten Teil des Füllerstoffs mit einen dritten Teil des Füllstoffs, der keine Komponente des Initiator-Systems enthält, homogen vermischt.

Bei den erfindungsgemäßen Dentalwerkstoffen fungiert der dritte Füllstoffanteil als Verdünner für die beiden initiator- bzw. aktivatorhaltigen Füllstoffanteile, so daß bei gewöhnlicher Lagerung des Füllstoffs keine Reaktion zwischen Initiator und Aktivator stattfindet. Beim Mischen des Füllstoffs mit dem Bindemittel werden die Bestandteile des Redox-Initiator-Systems durch das Bindemittel gelöst und die Redox-Reaktion initiiert. Durch das Mischen von Initiator und Aktivator mit je einem Teil des Füllstoffs wird eine gleichförmige Verteilung der Bestandteile und damit eine gleichmäßige Polymerisation des Dentalwerkstoffs gewährleistet.

Das Füllstoffgemisch enthält vorzugsweise 20 bis 90 Gew.-%, besonders bevorzugt 50 bis 90 Gew.-% und ganz besonders bevorzugt 70 bis 90 Gew.-% des dritten Füllstoffteils. Die Anteile des ersten und zweiten Füllstoffteils sind vorzugsweise etwa gleich groß.

Initiator und Aktivator werden vorzugsweise in einem solchen Verhältnis mit dem Füllstoff gemischt, daß der erste bzw. zweite Füllstoffteil bezogen auf die Summe der Massen aus Initiator bzw. Aktivator und Füllstoff 1 bis 20 Gew.-%, vorzugsweise 7 bis 15 Gew.-% des Initiators bzw. Aktivators enthält.

Das Verhältnis der einzelnen Füllstoffteile wird so gewählt, daß der Anteil der Redoxkomponenten vorzugsweise jeweils 0.01 bis 10 Gew.-%, besonders bevorzugt 0,02 bis 5 Gew.-%, ganz besonders bevorzugt 0,1 bis 5 Gew.-% bezogen auf die Gesamtmasse des Füllstoffs beträgt. Die am meisten bevorzugten Bereiche sind 0,2 bis 1 Gew.-% und insbesondere 0,5 bis 1 Gew.-% bezogen auf die Gesamtmasse des Füllstoffs.

Geeignete Redox-Initiator-Systeme werden in "Redox Polymerization", G.S. Misra und U.D.N. Bajpai, Proc. Polym. Sci., 8, 61-131 (1982) beschrieben.

Bevorzugte Initiatoren (oxidierende Stoffe) sind Kobalt(III)chlorid, tert.-Butylhydroperoxid, Eisen(III)chlorid, Hydroxylamin (abhängig von der Wahl des Aktivators), Perborsäure und ihre Salze, und Salze von Permanganat- oder Persulfatanionen. Wasserstoffperoxid kann ebenfalls verwendet werden, obwohl es bei gleichzeitiger Verwendung eines Photoinitiators zu Wechselwirkungen kommen kann.

Bevorzugte Aktivatoren (reduzierende Stoffe) sind Ascorbinsäure, Kobalt(II)-chlorid, Eisen(II)chlorid, Eisen (II)sulfat, Hydrazin, Hydroxylamin (abhängig von der Wahl des Initiators), Oxalsäure, Thioharnstoff und Salze von Dithionit- oder Sulfitanionen.

Besonders bevorzugte Aktivatoren sind Ascorbinsäure und Benzylphenylbarbitursäure (BPBS), besonders bevorzugte Initiatoren sind Benzoylperoxid (BPO) und Lauroylperoxid. Das am meisten bevorzugte Redox-System ist BPO/BPBS.

Neben dem Redoxinitiator können die erfindungsgemäßen Dentalwerkstoffe zusätzlich einen oder mehrere Photoinitiatoren enthalten. Bevorzugte Photoinitiatoren sind Benzoinether, Dialkylbenzilketale, Dialkoxyacetophenone, Acylphosphinoxide, $\alpha$-Diketone wie 9,10-Phenanthrenchinon, Diacetyl, Furil, Anisil, 4,4'-Dichlorbenzil und 4,4'-Dialkoxybenzil und insbesondere Campherchinon (vgl. J.P. Fouassier, J.F. Rabek (Herausgeber), Radiation Curing in Polymer Science and Technology, Vol. II, Elsevier Applied Science, London, New York 1993).

Bevorzugte Füllstoffe sind Quarz-, Glaskeramik- oder Glaspulver sowie Aluminium- und Siliciumoxidpulver, insbesondere Silikatgläser wie Bariumsilikat-, Li/Al-Silikatgläser, Ba/Al-Silikatgläser und Bariumgläser sowie Mischungen daraus. Besonders bevorzugte Füllstoffe sind ionenfreisetzende Gläser, insbesondere ionenfreisetzende Ba/Al-Silikatgläser.

Die Füllstoffe werden als Pulver mit einer Partikelgröße von vorzugsweise 0,1 bis 50 $\mu$m, insbesondere 1 bis 20 $\mu$m eingesetzt.

Darüber hinaus kann des Füllstoffgemisch zusätzlich Pigmente, Röntgenopazitätsmittel, vorzugsweise Ytterbiumtrifluorid, Thixotropiermittel wie pyrogene und/oder gefällte Kieselsäuren, Beschleuniger, beispielsweise Metallsalze und Komplexverbindungen wie Kupferacetat, Kupferacetylacetonat, Kupfersalicylat, Co-EDTA-Komplex, und weitere Hilfs- und Zusatzstoffe enthalten. Kieselsäuren werden üblicherweise in einer Menge von bis zu 20 Gew.-%, vorzugsweise 1 bis 10 Gew.-% verwendet.

Eine bevorzugte Füllstoffmischung enthält bezogen auf die Gesamtmasse an Füllstoff 40 bis 90 Gew.-% mindestens eines der oben genannten bevorzugten Füllstoffe, 0 bis 50 Gew.-% Röntgenopazitätsmittel und 0 bis 10 Gew.-% Thixotropiermittel.

Ein besonders bevorzugtes Füllstoffgemisch enthält 60 bis 80 Gew.-% Silikatglas, 20 bis 40 Gew.-% Ytterbiumtrifluorid und 1 bis 5 Gew.-% Fällungskieselsäure. Die Mischung enthält vorzugsweise außerdem 0,2 bis 1 Gew.-

% Benzoylperoxid und 0,2 bis 1 Gew.-% Benzylphenylbarbitursäure

Das Mischen der Bestandteile des Redox-Initiator-Systems und des Füllstoffs erfolgt durch Beschichten von Füllstoffpartikeln mit dem Initiator bzw. Aktivator, beispielsweise indem man den Füllstoff in einer Lösung des Initiators bzw. Aktivators aufschlämmt und anschließend das Lösungsmittel entfernt. Danach kann der Füllstoff gegebenenfalls getrocknet werden. Anschließend werden die unterschiedlichen Füllstoffanteile miteinander vermischt und gegebenenfalls weitere, trockene Hilfs- und Füllstoffe zugegeben. Der so erhaltene Füllstoff kann verpackt und gelagert werden.

Die Füllstoffe werden vorzugsweise silanisiert. Hierzu können übliche, käuflich erhältliche Silanisierungsmittel wie Vinyltriethoxysilan, Vinyltrimethoxysilan, Vinyl-tris(beta-methoxy-ethoxy)silan, gamma-Methacryloxypropyl-trimethoxy-silan (Silan A-174), gamma-Methacryloxypropyl-tris(2-methoxyethoxy)silan, gamma-Glycidoxypropyltrimethoxysilan, Vinyltriacetoxysilan, gamma-Mercaptopropyltrimethoxysilan,gamma-Aminopropyltriethoxysilan, N-beta-(Aminoethyl)-gamma-aminopropyltrimethoxysilan (Fa. Union Carbide) eingesetzt werden. Bevorzugt sind Silane ohne Amino- und Mercaptogruppen, besonders bevorzugt Silan A-174.

Ein besonderer Vorteil des erfindungsgemäßen Füllstoffgemischs ist, daß es oxidierende und reduzierende Stoffe in einem vorgegebenen Mengenverhältnis enthält und daß diese Komponenten nicht erst vom Anwender miteinander gemischt werden müssen. Die Füllstoffe sind einfach herzustellen, unterliegen nicht der Hydrolyse und weisen eine überraschend hohe Lagerstabilität auf.

Die Zusatz- und Hilfsstoffe sowie der Photoinitiator können entweder zu der Mischung der drei obligatorischen Füllstoffteile gegeben oder alternativ ihrerseits zuerst mit einem Teil des Füllstoffs gemischt und dann mit den drei genannten Füllstoffteilen vereinigt werden. Hierbei können die Hilfsstoffe ebenfalls wie beschrieben auf Füllstoffpartikel geschichtet werden. Alternativ können die Hilfsstoffe und der Photoinitiator auch dem Bindemittel zugegeben werden.

Die Komponenten des Redox-Initiator-Systems und gegebenenfalls die Hilfs- und Zusatzstoffe können auf unterschiedliche Bestandteile des Füllstoffs geschichtet werden. Alternativ können die verschiedenen Füllstoffkomponenten vereinigt und die Mischung danach in mehrere Anteile aufgeteilt werden, die dann mit den Komponenten des Redox-Initiator-Systems und gegebenenfalls den Zusatz- und Hilfsstoffen beschichtet und anschließend wieder vereinigt werden.

Das Bindemittel enthält mindestens ein polymerisierbares Monomer, Oligomer, Präpolymer und/oder Makromonomer. Als Makromere werden kurze Polymere mit einer terminalen, polymerisationsfähigen Gruppe bezeichnet. Oligomere sind aus mindestens 5, gewöhnlich aus 50 bis 300 Monomerbausteinen aufgebaut. Mit dem Begriff Präpolymer werden Oligomere und Polymere mit polymerisierbaren Gruppen zusammengefaßt.

Erfindungsgemäß geeignete Monomere werden in der US-A-5,554,665, Spalte 6 bis Spalte 8 beschrieben. Bevorzugte Monomere sind Methylmethacrylat, Isobutylmethacrylat, Butoxymethylmethacrylat, 2-Ethylhexylmethacrylat, Isodecylmethacrylat, Phenoxyethylmethacrylat, Ethylmethacrylat, Butylmethacrylat, Benzylmethacrylat, Phenylmethacrylat, Tetrahydrofurfurylmethacrylat, 1,4-Butandioldimethacrylat, 1,6-Hexandibldimethacrylat, 1,9-Nonandioldimethacrylat, Trimethylolpropantrimethacrylat, Pentaerythrittetramethacrylat, Dipentaerythritpentamethacrylat, Bis-[4-methacryloxy-2-hydroxypropyloxyphenyl]propan, Polyethylenglykolmethacrylate, beispielsweise auf Basis von Polyethylenglycol (PEG) 300, 400 oder 1000, Bisphenol-A-dimethacrylat und insbesondere Ethylenglykoldimethacrylat, Triethylenglykoldimethacrylat, Dipropylenglykoldimethacrylat, Bis-GMA (2,2-Bis-4-(3-methacryloxy-2-hydroxypropyl)-phenylpropan), 1,1,6-Trimethylhexamethylenurethandimethacrylat, Cyclohexylmethacrylat, Urethandimethacrylat (UD-MA, Reaktionsprodukt von Hydroxyethyl(meth)acrylat oder Hydroxypropyl(meth)acrylat mit 2,2,4-Trimethylhexyl-1,6-diisocyanat), Hydroxyethylmethacrylat (HEMA), Glycerinmono-, -di- und -trimethacrylat. Besonders bevorzugt sind Hydroxyethylmethacrylat und Glycerindimethacrylat (GDMA).

Bevorzugte Oligomere sind saure Oligomere, die neben Methacrylatgruppen Carboxylatgruppen enthalten, ganz besonders bevorzugt sind statistische Oligomere auf Basis von Acrylsäure und Glycidylmethacrylat.

Oligomere auf Basis von Acrylsäure und Glycidylmethacrylat werden vorzugsweise erhalten, indem man zunächst Acrylsäure mit einem Radikalüberträger (transfer agent) in einem Lösungsmittel mit einem Initiator für die radikalische Polymerisation teilpolymerisiert, d.h. zu Oligomeren mit 5 bis 40 Monomereinheiten umsetzt. Vorzugsweise werden n mol Acrylsäure pro mol Radikalüberträger eingesetzt, wobei die Zahl n vorzugsweise 5 bis 40, besonders bevorzugt 5 bis 20 und ganz besonders bevorzugt 8 bis 12 ist.

Als Lösungsmittel kommen vorzugsweise polare organische Lösungsmittel mit einem Siedepunkt über 50 °C in Betracht. Bevorzugt sind Alkohole wie Ethanol, Propanol und Butanol und insbesondere sek.-Butanol.

Als Radikalüberträger eignen sich Peroxide, Chloroform, Azoverbindungen, wie beispielsweise Azoisoburyronitril, und insbesondere Mercaptane, ganz besonders 2-Mercaptoethanol.

Bevorzugte Initiatoren sind Azoverbindungen wie Azoisobutyronitril und insbesondere Azobiscyanovaleriansäure.

Die Reaktionstemperatur kann in weiten Grenzen schwanken und liegt vorzugsweise im Bereich von 50 bis 120 °C, insbesondere 80 bis 100 °C.

Als Zwischenprodukt wird eine oligomere Acrylsäure erhalten, deren Endgruppen durch den verwendeten

Radikalüberträger bestimmt werden. Die Verwendung von 2-Mercaptoethanol ergibt beispielsweise Verbindungen mit 2-Hydroxyethylthioendgruppen gemäß der Formel

$$H-[-CH_2-CH(COOH)]_n-S-(CH_2)_2OH,$$

wobei n die oben angegebene Bedeutung hat.

Das Zwischenprodukt wird mit einem m-fachen, vorzugsweise 3- bis 40-fachen, besonders bevorzugt 3- bis 20-fachen und ganz besonders bevorzugt 3 bis 7-fachen molaren Überschuß an Glycidylmethacrylat bei 40 bis 90 °C, vorzugsweise 50 bis 70 °C, zweckmäßigerweise unter Normaldruck umgesetzt. In der Regel kann das Produkt aus der ersten Stufe ohne weitere Aufarbeitung direkt in die zweite Stufe eingesetzt werden. Das Endprodukt wird im allgemeinen nach dem Entfernen des Lösungsmittels erhalten. Die Reaktionsfolge führt zu statistischen Oligomeren mit einem Molekulargewicht von 1000 bis ca. 30 000, vorzugsweise 1200 bis 10 000, besonders bevorzugt 1200 bis 5000. Das Molekulargewicht der statistischen Oligomere hängt von der Kettenlänge des Zwischenprodukts ab. Es wird etwa die Hälfte der Carbonsäuregruppen verestert.

Das unter Verwendung von 2-Mercaptoethanol als Radikalüberträger erhaltene Produkt kann näherungsweise durch die Formel

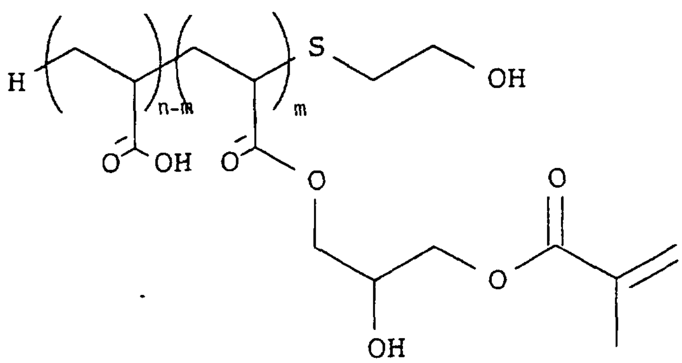

beschrieben werden, wobei die Gruppen -COOH und $-COOCH_2-CH(OH)-CH_2-O-CO-C(CH_3)=CH_2$ statistisch in der Kette von n Einheiten verteilt sind.

Bevorzugte Präpolymere werden in der WO 96/09332 offenbart. Hierbei handelt es sich um Block-Copolymere aus polymerisierbaren Oligo- und/oder Polyalkensäuren der allgemeinen Formel $A-[-B_x-C_y-D_z-]_n-E$, in der die Gruppen A und E gleich oder verschieden Wasserstoff, Halogenatome, Methacrylat-, Acrylat-, Allyl-, Vinyl-, Alkyl-, Aryl-, Alkoxy-, Aryloxy-, Carboxyl-, Amin-, Hydroxy-, Isocyanat-, Silyl- und/oder Siloxygruppen sind und als Endgruppen fungieren.

Das Oligomer/Polymer besteht aus mindestens zwei Blöcken (hier = Gruppen) von Sequenzen, wobei die eine Gruppe B ein Segment bestehend aus Sequenzen von Mono-, Di- und/oder Tricarbonsäuren als Monomerbausteine, deren Anhydriden, Salzen und/oder Derivaten dieser Säuren mit einer an sich bekannten Säureschutzgruppe ist, aus denen die Säure leicht freizusetzen ist. Die andere Gruppe C besteht aus Sequenzen von Mono-, Di- und/oder Tricarbonsäureestern und/oder deren Amiden sowie Isopren und/oder Butadienbausteinen als Monomerbausteine, wobei die Alkoholkomponente der Ester, sowie die Aminkomponente der Amide jeweils ungesättigt ist.

Eine dritte optionale Gruppe D besteht aus Sequenzen von Mono-, Di- und/oder Tricarbonsäuren als Monomerbausteine, deren Estern, Amiden und/oder Nitrilen, die keine Doppelbindungen enthalten.

Die Abfolge der Gruppen B, C und D innerhalb der Blöcke $[-B_x-C_y-D_z-]$ ist beliebig und kann für jedes n unterschiedlich sein.

Die in den Gruppen B; C und D in sequentieller Abfolge enthaltenen Monomerbausteine sind für ein bestimmtes n jeweils gleich, können aber für unterschiedliche n verschieden sein.

Die Indizes bedeuten n = 1 bis 10, x, y, z = 0 oder 4 bis 1000, wobei für mindestens ein n x mindestens 4 und y mindestens 4 sind und x, y, z für jedes n unterschiedlich sein können. Bevorzugt ist n = 1 bis 4, x und y mindestens 4 und z = 0 oder mindestens auch 4. Bevorzugt sind Präpolymere mit der Formel:

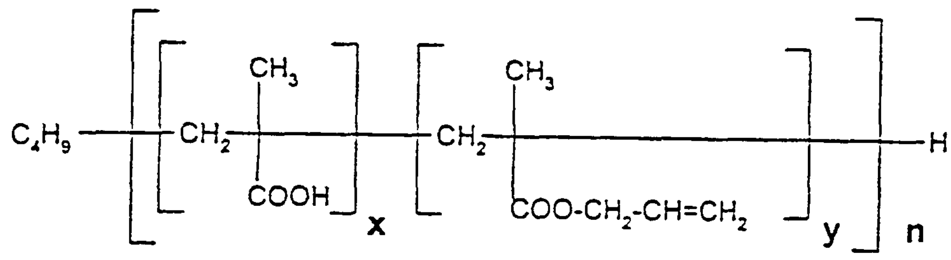

mit x = 180-200, y = 80-100; n = 1

Das Bindemittel ist vorzugsweise flüssig, besonders bevorzugt dünnflüssig. Zur Gewährleistung einer ausreichenden Fließfähigkeit und zur Erleichterung des Mischens mit dem Füllstoff kann das Bindemittel ein Lösungsmittel enthalten. Bevorzugte Lösungsmittel sind Alkohole, insbesondere Ethanol, Wasser, oder deren Gemische. Darüber hinaus können flüssige Monomere als Lösungsmittel dienen. Das am meisten bevorzugte Lösungsmittel ist Wasser. Der Lösungsmittelgehalt wird so gewählt, daß das Bindemittel die gewünschte Viskosität aufweist. Üblicherweise beträgt der Lösungsmittelanteil 0 bis 80 Gew.-%, besonders bevorzugt 10 bis 40 Gew.-%.
Die Bindemittel enthalten vorzugsweise eine Mischung von mindestens einem Monomer und mindestens einem Oligomer, Präpolymer und/oder Makromonomer.
Bevorzugt sind Bindemittel, die bezogen auf die Gesamtmasse des Bindemittels 5 bis 80 Gew.-% Monomer, 1 bis 40 Gew.-% Oligomer, Präpolymer oder Makromonomer und 10 bis 40 Gew.-% Lösungsmittel enthalten.
Ein besonders bevorzugtes Bindemittel enthält 10 bis 40 Gew.-% Hydroxyethylmethacrylat, 10 bis 40 Gew.-% Glycerindimethacrylat, 10 bis 40 Gew.-% eines statistischen Oligomers auf Basis von Acrylsäure, 2-Mercaptoethanol und Glycidylmethacrylat und 10 bis 30 Gew.-% Wasser.
Erfindungsgemäß sind solche Dentalmaterialien besonders bevorzugt, die neben einem polymerisierbaren Bindemittel mit organischen Säuregruppen einen oder mehrere ionenfreisetzende Füllstoffe enthalten. Die Härtung dieser Dentalwerkstoffe erfolgt einerseits durch eine Säure-Base-Reaktion zwischen Bindemittel und Füllstoff und andererseits durch radikalische Polymerisation des Bindemittels. Die ionische Reaktion wird durch die Verwendung von Wasser als Lösungsmittel begünstigt.
Bei Dentalzementen werden Bindemittel und Füllstoff vorzugsweise in einem Verhältnis von 1,5 : 1 bis 3 : 1, besonders bevorzugt 2 : 1 bis 2,5 : 1 gemischt. Bei Füllstoffen beträgt das Verhältnis von Bindemittel zu Füllstoff vorzugsweise 0,5 : 1 bis 1,5 : 1.
Die Mischung der Komponenten erfolgt kurz vor der Anwendung. Die Bearbeitungszeit beträgt in der Regel 1 bis 5 Minuten, vorzugsweise 2 bis 3 Minuten.
Die Dentalmaterialien können in transparenter oder eingefärbter Form verwendet werden.
Im folgenden wird die Erfindung anhand von Ausführungsbeispielen erläutert.

## Beispiel 1

**Synthese eines statistischen Oligomeren auf Basis von Acrylsäure, 2-Mercaptoethanol und Glycidylmethacrylat**

350 ml sek.-Butanol wurden über einen Zeitraum von 60 Minuten bei 95 °C unter heftigem Rühren gleichzeitig mit 288,8 g (4 mol) Acrylsäure und einer Lösung von 31,2 g (0,4 mol) Mercaptoethanol und 5,6 g (20 mmol) Azobiscyanovaleriansäure in 250 ml sek.-Butanol versetzt. Um den vollständigen Zerfall des Initiators zu gewährleisten wurde anschließend für 16 Stunden ei 90 °C gerührt. Das Zwischenprodukt läßt sich durch die Formel

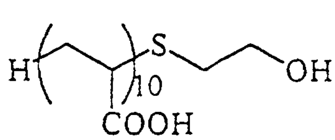

beschreiben.

$^1$H-NMR ($CDCl_3$/DMSO-$D_6$): δ = 1, 4 9 - 1,61 (br, $CH_{2,\ Hauptkette}$), 2,15-2 , 62 (br, $CH_{Hauptkette}$), 2,71 (t, $CH_2S$), 3,71 (t, $CH_2O$) und 11,97 (br, Säure) ppm.

IR (Film): 3580 - 2400 (s und br), 2956 (s), 1709 (s), 1411 (m) und 1238 $cm^{-1}$.

Danach wurde das Reaktionsgemisch ohne weitere Aufreinigung mit 227,4 g (1,6 mol) GMA und 80 mg Hydrochinonmonomethylether (MeHQ) als Stabilisator versetzt und für 2 Tage bei 60 °C gerührt. Die Abnahme der GMA-Konzentration wurde mittels HPLC verfolgt. Bei der Reaktion wurden 50 % der Carbonsäurereste mit Glycidylmethacrylat umgesetzt. Das Molekulargewicht des erhaltenen Oligomeren betrug etwa 1500. Das Produkt läßt sich näherungsweise durch die Formel

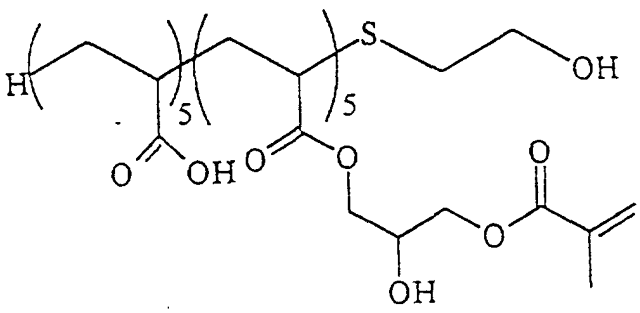

beschreiben, wobei die Verteilung der Gruppen -COOH und $-COOCH_2-CH(OH)-CH_2-O-CO-C(CH_3)=CH_2$ in der Kette von 10 Einheiten statistisch ist.

$^1$H-NMR ($CDCl_3$/DMSO-$D_6$): δ = 1,43 - 1,68 (br, $CH_{2,\ Hauptkette}$), 1,93 (s, $CH_3$), 2,15 - 2,58 (br, $CH_{Hauptkette}$), 2,76 (t, $CH_2S$), 3,70 - 4,40 (br, CHO und $CH_2O$), 5,64 und 6,15 (2 s, $=CH_2$) und 12,18 (br, Säure) ppm.

IR (Kap.): 3500 - 2400 (s und br), 2960 (s), 1712 (s), 1635 (m), 1453 (m), 1298 (s) und 1161 (s) $cm^{-1}$.

Danach wurde die Mischung mit 600 g HEMA versetzt und das Lösungsmittel unter Einleiten von Luft im Vakuum (180 mbar) bei maximal 55 °C am Rotationsverdampfer entfernt.

## Beispiel 2

### Herstellung eines Dentalmaterials

1. Bindemittel

Es wird eine Mischung der folgenden Komponenten hergestellt:

| | |
|---|---|
| 19,8 Gew.-% | Wasser (deionisiert) |
| 27,9 Gew.-% | Hydroxyethylmethacrylat (HEMA) |
| 27,6 Gew.-% | Glycerin-1,3-dimethacrylat (GDMA) |
| 24,7 Gew.-% | Oligomer aus Beispiel 1 (gelöst in HEMA, der HEMA-Anteil ist in den 27.9 Gew.-% bereits enthalten) |

2. Füllstoff

2.1 Silanisierung des Füllstoffs

95,24 Gew.-% eines Fluor-Calcium-Aluminium-Silikatglaspulvers (23,1 Gew.-% $SiO_2$; 21,6 Gew.-% $Al_2O_3$ ; 16,4 Gew.-% CaO; 8,7 Gew.-% $P_2O_5$ ; 1,9 Gew.-% $Na_2O$ ; 11,2 Gew.-% BaO ; 17,1 Gew.-% F; Ionomerglas SP-2034, Firma Specialty Glass) mit einer mittleren Teilchengröße von 7 µm wurden in einem geeigneten Behälter gleichmäßig mit 1,90 Gew.-% deionisiertem Wasser benetzt und anschließend für 15 Minuten gut homogenisiert. Danach wurde die Mischung vorsichtig mit 2,86 Gew.-% gamma-Methacryloxypropyl-trimethoxy-silan (Silan A-174, Firma Union Carbide) versetzt, wiederum für 15 Minuten homogenisiert und dann in einem verschlossenen Behälter für 24 Stunden stehengelassen. Bei der Zugabe des Wassers bzw. des Silans ist auf eine möglichst gleichmäßige Verteilung zu achten, um eine Klumpenbildung zu verhindern.

Das silanisierte Ionomerglas wird durch ein Sieb mit einer Maschenweite von 125 µm gesiebt, bei 50 °C

getrocknet und danach durch ein Nylonsieb mit einer Maschenweite von 90 µm gesiebt.

2.2 Beschichten des Füllstoffs mit Initiator

In einem metallfreien Behälter wurden unter Rühren mit einem metallfreien Rührer 2,13 Gew.-% Benzoylperoxid (BPO, 76%ig, mit 24 Gew.-% Wasser angefeuchtet) in 81,56 Gew.-% Essigsäureethylester gelöst. Anschließend wurden 16,31 Gew.-% des silanisierten Ionomerglases zugegeben und in der Lösung aufgeschlämmt. Danach wurde das Lösungsmittel unter kräftigem Rühren der Suspension im Vakuum bis zur Trockene abgedampft. Das Abdampfen des Lösungsmittels sollte möglichst langsam durchgeführt werden, um eine gleichmäßige Beschichtung der Glaspartikel mit dem BPO zu erzielen. Der Kuchen aus beschichtetem Pulver wurde aufgebrochen und vorsichtig von Hand zerkleinert. Nach dem Trocknen im Vakuum wurde das erhaltene Pulver durch ein Nylonsieb mit einer Maschenweite von 90 µm gesiebt.

2.3 Beschichten des Füllstoffs mit Aktivator

In einem metallfreien Behälter mit metallfreiem Rührer wurden 1,64 Gew.-% Benzylphenylbarbitursäure (BPBS) in 81,97 Gew.-% Essigsäureethylester gelöst. Anschließend wurden 16,39 Gew.-% des silanisierten Ionomerglaspulvers zugegeben und in der Lösung aufgeschlämmt. Danach wurde das Lösungsmittel unter kräftigem Rühren der Suspension im Vakuum zur Trockene eingedampft. Das Abdampfen des Lösungsmittels sollte möglichst langsam erfolgen, um eine gleichmäßige Beschichtung der Oberfläche der Glaspartikel mit der Benzylphenylbarbitursäure zu erzielen. Der Kuchen aus beschichtetem Pulver wurde aufgebrochen und vorsichtig von Hand zerkleinert. Nach dem Trocknen im Vakuum wurde das Pulver durch ein Nylonsieb mit einer Maschenweite von 90 µm gesiebt.

2.4 Mischen der Füllstoffanteile

In einem Rhönradmischer (Fa. Engelsmann) wurde unter Kühlung bei einer Temperatur von nicht mehr als 30 °C eine homogene Mischung der folgenden Komponenten hergestellt:

| | |
|---|---|
| 59,0 Gew.-% | silanisiertes Ionomerglas |
| 8,0 Gew.-% | Benzoylperoxid-beschichtetes Ionomerglas |
| 6,0 Gew.-% | Benzylphenylbarbitursäure-beschichtetes Ionomerglas |
| 25,0 Gew.-% | $YbF_3$ (Fa. Rhone Poulenc) |
| 2,00 Gew.-% | hochdisperse Kieselsäure mit einer BET Oberfläche von 392 $m^2$/g (HDK T 40, Firma Wacker) |

3. Vereinigen von Bindemittel und Füllstoff

Bindemittel und Füllstoff wurden im Gewichtsverhältnis 1 : 2,25 auf einer Glasplatte mit einem Spatel vermischt.

Nach dem Härten wies das Dentalmaterial folgende physikalische Eigenschaften auf:

| | |
|---|---|
| Elastizitätsmodul [1] | 3300 - 3800 MPa |
| Biegefestigkeit [1] | 40 - 50 MPa |
| Verarbeitungszeit | 120 ± 30 sek bei 23° C |
| Aushärtungszeit | 360 - 420 sek bei 23° C |
| Expansion [2] | 0,4% |

1 : Bestimmt gemäß ISO Norm 4049 (1988) nach den oben angegebenen Zeiten.

2 : Nach 4wöchiger Lagerung in 0,8%iger NaCl-Lösung bei 37° C

Die Materialien zeichnen sich bei guter Biegefestigkeit und zufriedenstellendem E-Modul durch eine besonders geringe Expansion nach mehrwöchiger Wasserlagerung aus.

**Patentansprüche**

**1.** Verfahren zur Herstellung eines Dentalfüllstoffgemischs enthaltend ein Redox-Initiator-System für die radikalische

Polymerisation, das einen Initiator und einen Aktivator umfaßt, **dadurch gekennzeichnet, daß** man einen ersten Teil des Füllstoffs mit dem Initiator beschichtet, man einen zweiten Teil des Füllstoffs mit dem Aktivator beschichtet und man den ersten und zweiten Teil des Füllstoffs mit einem dritten Teil des Füllstoffs homogen vermischt, der keine Komponente des Initiator-Systems enthält.

**2.** Verfahren gemäß Anspruch 1, **dadurch gekennzeichnet, daß** der Anteil des dritten Füllstoffteils 20 bis 90 Gew.-% bezogen auf die Gesamtmasse des Füllstoffs beträgt.

**3.** Verfahren gemäß Anspruch 1 oder 2, **dadurch gekennzeichnet, daß** der erste Füllstoffteil 1 bis 20 Gew.-% des Initiators und/oder der zweite Füllstoffteil 1 bis 20 Gew.-% des Aktivators jeweils bezogen auf die Summe der Massen von Initiator bzw. Aktivator und Füllstoff enthält.

**4.** Verfahren gemäß einem der Ansprüche 1 bis 3, **dadurch gekennzeichnet, daß** der Füllstoff jeweils 0,01 bis 10 Gew.-% Initiator bzw. Aktivator bezogen auf die Gesamtmasse des Füllstoffs enthält.

**5.** Verfahren gemäß einem der Ansprüche 1 bis 4, **dadurch gekennzeichnet, daß** der Initiator Benzoylperoxid und/oder Lauroylperoxid ist.

**6.** Verfahren gemäß einem der Ansprüche 1 bis 5, **dadurch gekennzeichnet, daß** der Aktivator Ascorbinsäure und/oder Benzylphenylbarbitursäure ist.

**7.** Verfahren gemäß einem der Ansprüche 1 bis 6, **dadurch gekennzeichnet, daß** man als Füllstoff Quarz-, Glaskeramik-, Glaspulver, Aluminium- und/oder Siliciumoxid verwendet.

**8.** Verfahren gemäß Anspruch 7, **dadurch gekennzeichnet, daß** der Füllstoff mindestens ein Silikatglas enthält.

**9.** Verfahren gemäß Anspruch 8, **dadurch gekennzeichnet, daß** der Füllstoff Bariumglas-, Bariumsilikatglas-, Li/Al-Silikatglas- und oder Ba/Al-Silikatglaspulver enthält.

**10.** Verfahren gemäß einem der Ansprüche 7 bis 9, **dadurch gekennzeichnet, daß** der Füllstoff zusätzlich Pigmente, Röntgenopazitätsmittel, Thixotropiermittel und/oder Beschleuniger enthält.

**11.** Verfahren gemäß Anspruch 10, **dadurch gekennzeichnet, daß** der Füllstoff bezogen auf die Gesamtmasse des Füllstoffs 60 bis 80 Gew.-% Silikatglas 20 bis 40 Gew.-% Ytterbiumtrifluorid, 1 bis 5 Gew.-% Fällungskieselsäure enthält.

**12.** Verfahren gemäß Anspruch 11, **dadurch gekennzeichnet, daß** der Füllstoff 0,2 bis 1 Gew.-% Benzoylperoxid und 0,2 bis 1 Gew.-% Benzylphenylbarbitursäure enthält.

**13.** Füllstoffgemisch erhältlich gemäß dem Verfahren nach einem der Ansprüche 1 bis 12.

**14.** Verfahren zur Herstellung eines Dentalwerkstoffs, **dadurch gekennzeichnet, daß** man mindestens ein polymerisierbares Bindemittel und mindestens einen Füllstoff gemäß Anspruch 13 miteinander mischt.

**15.** Verfahren Anspruch 14, **dadurch gekennzeichnet, daß** man als Bindemittel Methylmethacrylat, Isobutylmethacrylat, Butoxymethylmethacrylat, 2-Ethylhexylmethacrylat, Isodecylmethacrylat, Phenoxyethylmethacrylat, Ethylmethacrylat, Butylmethacrylat, Benzylmethacrylat, Phenylmethacrylat, Tetrahydrofurfurylmethacrylat, 1,4-Butandioldimethacrylat, 1,6-Hexandioldimethacrylat, 1,9-Nonandioldimethacrylat, Trimethylolpropantrimethacrylat, Pentaerythrittetramethacrylat, Dipentaerythritpentamethacrylat, Bis-[4-methacryloxy-2-hydroxypropyloxyphenyl]propan, Polyethylenglykolmethacrylate, Bisphenol-A-dimethacrylat, Ethylenglykoldimethacrylat, Triethylenglykoldimethacrylat, Dipropylenglykoldimethacrylat, Bis-GMA (2,2-Bis-4-(3-methacryloxy-2-hydroxypropyl)-phenylpropan), 1,1,6-Trimethylhexamethylenurethandimethacrylat, Cyclohexylmethacrylat, Urethandimethacrylat (UDMA, Reaktionsprodukt von Hydroxyethyl (meth)acrylat oder Hydroxypropyl(meth)acrylat mit 2,2,4-Trimethylhexyl-1,6-diisocyanat), Hydroxyethylmethacrylat (HEMA), Glycerinmono-, -di- (GDMA) und -trimethacrylat, Hydroxyethylmethacrylat, Glycerindimethacrylat, ein saures, polymerisierbares Oligomer und/oder ein saures, polymerisierbares Blockcopolymer verwendet.

**16.** Verfahren gemäß Anspruch 15, **dadurch gekennzeichnet, daß** man als Bindemittel Hydroxyethylmethacrylat,

Glycerindimethacrylat, ein statistisches Oligomer auf der Basis von Acrylsäure, 2-Mercaptoethanol und Glycidylmethacrylat oder Mischungen daraus verwendet.

**17.** Verfahren gemäß einem der Ansprüche 14 bis 16, **dadurch gekennzeichnet, daß** das Bindemittel flüssig ist.

**18.** Verfahren gemäß Anspruch 17, **dadurch gekennzeichnet, daß** das Bindemittel ein Lösungsmittel enthält.

**19.** Verfahren gemäß Anspruch 18, **dadurch gekennzeichnet, daß** das Bindemittel Wasser enthält.

**20.** Verfahren gemäß Anspruch 19, **dadurch gekennzeichnet, daß** das Bindemittel bezogen auf die Gesamtmasse des Bindemittels 10 bis 40 Gew.-% Hydroxyethylmethacrylat, 10 bis 40 Gew.-% Glycerindimethacrylat, 10 bis 40 Gew.-% eines statistischen Oligomers auf der Basis von Acrylsäure, 2-Mercaptoethanol und Glycidylmethacrylat und 10 bis 30 Gew.-% Wasser enthält.

**21.** Verfahren gemäß einem der Ansprüche 16 bis 20, **dadurch gekennzeichnet, daß** das statistisches Oligomer bezogen auf 1 mol 2-Mercaptoethanoleinheiten 8 bis 12 mol Acrylsäureeinheiten und 5 bis 40 mol Glycidylmethacrylateinheiten enthält.

**22.** Verfahren gemäß Anspruch 21, **dadurch gekennzeichnet, daß** das statistisches Oligomer ein mittleres zahlenmäßiges Molekulargewicht von 1000 bis 30000 aufweist.

**23.** Dentalwerkstoff erhältlich gemäß dem Verfahren nach einem der Ansprüche 14 bis 22.

**24.** Dentalwerkstoff gemäß Anspruch 23, **dadurch gekennzeichnet, daß** er zusätzlich einen Initiator für die Photopolymerisation enthält.

## Claims

**1.** Process for the manufacture of a dental filler mixture that comprises a redox initiator system for radical polymerisation, which includes an initiator and an activator, **characterised in that** a first portion of the filler is coated with the initiator, a second portion of the filler is coated with the activator, and the first and second portions of the filler are homogeneously blended with a third portion of the filler, which does not comprise any component of the initiator system.

**2.** Process according to Claim 1, **characterised in that** the proportion of the third filler part is 20 to 90 wt.% of the overall weight of the filler.

**3.** Process according to Claim 1 or 2, **characterised in that** the first filler part contains 1 to 20 wt.% of the initiator, and/or the second filler part contains 1 to 20 wt.% of the activator, in each case, based on the sum of the weights of initiator or activator and filler.

**4.** Process according to one of Claims 1 to 3, **characterised in that** the filler comprises 0.01 to 10 wt.% each of initiator or activator, based on the overall weight of the filler.

**5.** Process according to one of Claims 1 to 4, **characterised in that** the initiator is benzoyl peroxide and/or lauryl peroxide.

**6.** Process according to one of Claims 1 to 5, **characterised in that** the activator is ascorbic acid and/or benzylphenylbarbituric acid.

**7.** Process according to one of Claims 1 to 6, **characterised in that** quartz powder, ceramic glass powder, glass powder, aluminium oxide and/or silicon dioxide are used as the filler.

**8.** Process according to Claim 7, **characterised in that** the filler comprises at least one silicate glass.

**9.** Process according to Claim 8, **characterised in that** the filler comprises barium glass powder, barium silicate glass powder, Li/Al-silicate glass powder and/or Ba/Al-silicate glass powder.

**10.** Process according to one of Claims 7 to 9, **characterised in that** the filler additionally comprises pigments, X-ray opacity agents, thixotropic agents and/or accelerators.

**11.** Process according to Claim 10, **characterised in that** the filler comprises 60 to 80 wt.% silicate glass, 20 to 40 wt.% ytterbium trifluoride, and 1 to 5 wt.% precipitated silica, based on the overall weight of the filler.

**12.** Process according to Claim 11, **characterised in that** the filler comprises 0.2 to 1 wt.% of benzoyl peroxide and 0.2 to 1 wt.% benzylphenylbarbituric acid.

**13.** A filler mixture obtainable according to the process of one of Claims 1 to 12.

**14.** Process for manufacturing a dental material **characterised in that** at least one polymerisable binder and at least one filler according to Claim 13 are mixed with one another.

**15.** Process of Claim 14, **characterised in that** methyl methacrylate, isobutyl methacrylate, butoxymethyl methacrylate, 2-ethylhexyl methacrylate, isodecyl methacrylate, phenoxyethyl methacrylate, ethyl methacrylate, butyl methacrylate, benzyl methacrylate, phenyl methacrylate, tetrahydrofurfuryl methacrylate, 1,4-butanediol dimethacrylate, 1,6-hexanediol dimethacrylate, 1,9-nonanediol dimethacrylate, trimethylolpropane trimethacrylate, pentaerythritol tetramethacrylate, dipentaerythritol pentamethacrylate, bis(4-methacryloxy-2-hydroxypropyloxyphenyl)propane, polyethylene glycol methacrylate, bisphenol-A dimethacrylate, ethylene glycol dimethacrylate, triethylene glycol dimethacrylate, dipropylene glycol dimethacrylate, bis-GMA (2,2-bis-4-(3-methacryloxy-2-hydroxypropyl)-phenyl propane), 1,1,6-trimethylhexamethyleneurethane dimethacrylate, cyclohexyl methacrylate, urethane dimethacrylate (UDMA, reaction product of hydroxyethyl (meth)acrylate or hydroxypropyl (meth)acrylate with 2,2,4-trimethylhexyl-1,6-diisocyanate), hydroxyethyl methacrylate (HEMA), glycerine mono-, -di-(GDMA) and -trimethacrylate, hydroxyethyl methacrylate, glycerine dimethacrylate, an acidic, polymerisable oligomer and/or an acidic, polymerisable block copolymer are used as the binder.

**16.** Process according to Claim 15, **characterised in that** hydroxyethyl methacrylate, glycerine dimethacrylate, a statistical oligomer based on acrylic acid, 2-mercaptoethanol, glycidyl methacrylate, or mixtures thereof are used as the binder.

**17.** Process according to one of Claims 14 to 16, **characterised in that** the binder is liquid.

**18.** Process according to Claim 17, **characterised in that** the binder comprises a solvent.

**19.** Process according to Claim 18, **characterised in that** the binder comprises water.

**20.** Process according to Claim 19, **characterised in that** the binder comprises 10 to 40 wt.% hydroxyethyl methacrylate, 10 to 40 wt.% glycerine dimethacrylate, 10 to 40 wt.% of a statistical oligomer based on acrylic acid, 2-mercaptoethanol and glycidyl methacrylate and 10 to 30 wt.% water, based on the overall weight of the binder.

**21.** Process according to one of Claims 16 to 20, **characterised in that** the statistical oligomer comprises 8 to 12 moles acrylic acid units and 5 to 40 moles glycidyl methacrylate units, based on 1 mole of 2-mercaptoethanol units.

**22.** Process according to Claim 21, **characterised in that** the statistical oligomer has a mean number-average molecular weight of 1000 to 30 000.

**23.** Dental material obtainable according to the process of one of Claims 14 to 22.

**24.** Dental material according to Claim 23, **characterised in that** it additionally comprises a photopolymerisation initiator.

## Revendications

**1.** Procédé de préparation d'un mélange de matériaux de charge dentaires, contenant un système initiateur redox pour la polymérisation radicalaire, qui comprend un initiateur et un activateur, **caractérisé en ce que** l'on enduit une première partie du matériau de charge avec l'initiateur, **en ce qu'**on enduit une deuxième partie du matériau

de charge avec l'activateur, et qu'on mélange de façon homogène la première et la deuxième partie du matériau de charge avec une troisième partie du matériau de charge qui ne contient aucun composant du système initiateur.

2. Procédé selon la revendication 1, **caractérisé en ce que** la quantité de la troisième partie du matériau de charge est comprise entre 20 et 90 % en poids, rapportés à la masse totale de la charge.

3. Procédé selon la revendication 1 ou 2, **caractérisé en ce que** la première partie du matériau de charge contient de 1 à 20 % en poids d'initiateur et/ou **en ce que** la deuxième partie du matériau de charge contient 1 à 20 % en poids d'activateur, exprimés dans chaque cas par rapport à la somme des masses de l'initiateur, respectivement de l'activateur, et du matériau de charge.

4. Procédé selon l'une des revendications 1 à 3, **caractérisé en ce que** le matériau de charge contient respectivement de 0,01 à 10 % en poids d'initiateur et, respectivement d'activateur, par rapport à la masse totale du matériau de charge.

5. Procédé selon l'une des revendications 1 à 4, **caractérisé en ce que** l'initiateur est du peroxyde de benzoyle et/ou du peroxyde de lauroyle.

6. Procédé selon l'une des revendications 1 à 5, **caractérisé en ce que** l'activateur est de l'acide ascorbique et/ou de l'acide benzylphénylbarbiturique.

7. Procédé selon l'une des revendications 1 à 6, **caractérisé en ce que** l'on utilise à titre de matériau de charge, une poudre de quartz, de céramique de verre, ou de verre, de ou de l'oxyde d'aluminium et/ou de silicium.

8. Procédé selon la revendication 7, **caractérisé en ce que** le matériau de charge contient au moins un verre de silicate.

9. Procédé selon la revendication 8, **caractérisé en ce que** le matériau de charge contient de la poudre de verre de baryum, de verre de silicate de baryum, de verre de silicate de Li/Al, et/ou de verre de silicate de Ba/Al.

10. Procédé selon l'une des revendications 7 à 9, **caractérisé en ce que** le matériau de charge contient, en plus des pigments, des agents d'opacification aux rayons X, des promoteurs de thixotropie et/ou des accélérateurs.

11. Procédé selon la revendication 10, **caractérisé en ce que** la charge contient de 60 à 80 % en poids de verre de silicate, de 20 à 40 % en poids de trifluorure d'ytterbium, de 1 à 5 % en poids d'acide silicique de précipitation, rapportés à la masse totale de la charge.

12. Procédé selon la revendication 11, **caractérisé en ce que** le matériau de charge contient de 0,2 à 1 % en poids de peroxyde de benzoyle et de 0,2 à 1 % en poids d'acide benzylphénylbarbiturique.

13. Mélange de matériau de charge susceptible d'être obtenu selon le procédé de l'une des revendications 1 à 12.

14. Procédé de préparation d'un matériau dentaire, **caractérisé en ce que** l'on mélange ensemble au moins un liant polymérisable et au moins un matériau de charge selon la revendication 13.

15. Procédé selon la revendication 14, **caractérisé en ce que** l'on utilise à titre liant du méthacrylate de méthyle, du méthacrylate d'isobutyle, du méthacrylate de butoxyméthyle, du méthacrylate de 2-éthylhexyle, du méthacrylate d'isodécyle, du méthacrylate de phénoxyéthyle, du méthacrylate d'éthyle, du méthacrylate de butyle, du méthacrylate de benzyle, du méthacrylate de phényle, du méthacrylate de tétrahydrofurfuryle, du diméthacrylate de 1,4-butanediol, du diméthacrylate de 1,6-hexanediol, du diméthacrylate de 1,9-nonanediol, du triméthacrylate de triméthylolpropane, du tétraméthacrylate de dipentaerytritol, du pentaméthacrylate de dipentaerytritol, du bis-[4-méthacryloxy-2-hydroxypropyloxyphényl]propane, du méthacrylate de polyéthylèneglycol, du diméthacrylate de bisphénol A, du diméthacrylate d'éthylèneglycol, du diméthacrylate de triéthylèneglycol, du diméthacrylate de dipropylèneglycol, du bis-GMA (2,2-bis-4-(3-méthacryloxy-2-hydroxypropyl)-phénylpropane), du diméthacrylate de 1,1,6-triméthylhexaméthylèneuréthane, du méthacrylate de cyclohexyle, du diméthacrylate d'uréthane (UDMA, produit de réaction du (méth)acrylate d'hydroxyéthyle ou du (méth)acrylate d'hydroxypropyle avec le 2,2,4-triméthylhexyl-1,6-diisocyanate), du méthacrylate d'hydroxyéthyle (HEMA), du mono-, di- (GDMA) et triméthacrylate de glycérol, du méthacrylate d'hydroxyéthyle, du diméthacrylate de glycérol, et/ou un copolymère séquencé, acide

polymérisable.

**16.** Procédé selon la revendication 15, **caractérisé en ce que** l'on utilise à titre de liant du méthacrylate d'hydroxyéthyle, du diméthacrylate de glycérol, un oligomère statistique à base d'acide acrylique, de 2-mercaptoéthanol et de méthacrylate de glycidyle ou des mélanges de ces composés.

**17.** Procédé selon l'une des revendications 14 à 16, **caractérisé en ce que** le liant est liquide.

**18.** Procédé selon la revendication 17, **caractérisé en ce que** le liant contient un solvant.

**19.** Procédé selon la revendication 18, **caractérisé en ce que** le liant contient de l'eau.

**20.** Procédé selon la revendication 19, **caractérisé en ce que** le liant, contient, par rapport, à la masse totale du liant, de 10 à 40 % en poids de méthacrylate d'hydroxyéthyle, de 10 à 40 % en poids de diméthacrylate de glycérol, 10 à 40 % en poids d'un oligomère statistique à base d'acide acrylique, de 2-mercaptoéthanol et de méthacrylate de glycidyle, et de 10 à 30 % en poids d'eau.

**21.** Procédé selon l'une des revendications 16 à 20, **caractérisé en ce que** l'oligomère statistique contient, rapportées à 1 mol d'unités de 2-mercaptoéthanol, de 8 à 12 mol d'unités d'acide acrylique et 5 à 40 mol d'unités de méthacrylate de glycidyle.

**22.** Procédé selon la revendication 21, **caractérisé en ce que** l'oligomère statistique a un poids moléculaire moyen en nombre de 1000 à 30000.

**23.** Matériau dentaire susceptible d'être obtenu selon le procédé de l'une des revendications 14 à 22.

**24.** Matériau dentaire selon la revendication 23, **caractérisé en ce qu'**il contient en plus un initiateur de photopolymérisation.